(19) Europäisches Patentamt / European Patent Office / Office européen des brevets

(11) **EP 3 853 749 B1**

(12) **EUROPEAN PATENT SPECIFICATION**

(45) Date of publication and mention of the grant of the patent:
**25.12.2024 Bulletin 2024/52**

(21) Application number: **18933775.1**

(22) Date of filing: **18.09.2018**

(51) International Patent Classification (IPC):
*G06F 21/32* (2013.01)     *H04W 12/68* (2021.01)
*H04L 9/40* (2022.01)     *G06N 3/08* (2023.01)
*G06F 21/44* (2013.01)     *B25J 13/02* (2006.01)
*B25J 13/00* (2006.01)     *A61B 34/00* (2016.01)
*H04L 67/131* (2022.01)     *H04L 67/025* (2022.01)
*H04W 12/06* (2021.01)     *G06F 3/01* (2006.01)

(52) Cooperative Patent Classification (CPC):
**G06F 21/32; A61B 34/75; B25J 13/00; B25J 13/02;**
**G06F 21/44; G06N 3/08; H04L 63/083;**
**H04L 63/0861; H04W 12/06; H04W 12/68;**
A61B 34/35; A61B 34/74; G06F 3/011; H04L 67/025;
H04L 67/131

(86) International application number:
**PCT/FI2018/050677**

(87) International publication number:
**WO 2020/058561 (26.03.2020 Gazette 2020/13)**

(54) **APPARATUS AND METHOD FOR AUTHENTICATING A USER**

VORRICHTUNG UND VERFAHREN ZUR AUTHENTIFIZIERUNG EINES BENUTZERS

APPAREIL ET PROCÉDÉ D'AUTHENTIFICATION D'UN UTILISATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.07.2021 Bulletin 2021/30**

(73) Proprietor: **Nokia Technologies Oy**
**02610 Espoo (FI)**

(72) Inventors:
• **MICHE, Yoan Jean Claude**
**01800 Klaukkala (FI)**
• **KALLIOLA, Aapo**
**00710 Helsinki (FI)**
• **OLIVER, Ian Justin**
**01150 Söderkulla (FI)**

(74) Representative: **Sayer, Robert David**
**Venner Shipley LLP**
**200 Aldersgate**
**London EC1A 4HD (GB)**

(56) References cited:
WO-A1-2016/127006     WO-A2-2016/127005
US-A1- 2002 120 361     US-A1- 2007 094 208
US-A1- 2010 217 991     US-A1- 2017 282 365
US-A1- 2017 361 468

• OROZCO, M. ET AL.: "Automatic identification of participants in haptic systems", 2005 IEEE INSTRUMENTATION AND MEASUREMENT TECHNOLOGY CONFERENCE PROCEEDINGS, 16 May 2005 (2005-05-16), pages 888 - 892, XP001509070, [retrieved on 20190524]

## Description

### Field

Embodiments relate to an apparatus and method for authenticating a user, for example a user controlling a remote device.

### Background

Computer systems, including virtual reality (VR) systems, allow for remote execution of some actions, for example controlling a remote device such as a robot. A user can operate, from a first location, a remote device located at a second location by means of a link such as a communications network. In such a situation, there may be a need for a user to be positively authenticated in order to control the remote device. WO2016/127006 discloses an access control method with neuro and neuro mechanical finterprints. US2007/094208 discloses a method and system for identifying client users from user behavioural data. Orozco, M. et al: "Automatic identification of participants in haptic systems" 2005, IEEE Instrumentation and Measurement Technology Conference Proceedings, 16 May 2005, p 888 - 892 discloses biometric systems for identifying users. US2017/361468 discloses systems and methods to control an autonomous mobile robot.

### Summary

The invention is set forth in the claims. In a first aspect, the specification describes an apparatus comprising: means for receiving a control signal from a user-operated control apparatus for controlling a remote apparatus, wherein the control signal is a composite signal comprising a command signal for controlling the remote apparatus and a user noise signal; means for extracting the user noise signal from the received control signal by subtracting from the control signal one or more known command signals for possible control actions of the remote apparatus; means for determining if the user noise signal meets one or more predetermined criteria; and means for authenticating a user of the control apparatus at least partially based on the user noise signal determination.

The apparatus may further comprise means for identifying a user from whom the control signal is received, and wherein determining if the user noise signal meets the predetermined criteria includes means for comparing the user noise signal with stored user noise signature data corresponding to the identified user.

The means for identifying the user may comprise means for receiving user-input prior to receiving the control signal.

The received user-input may comprise one or more of a password, fingerprint, or other biometric data.

The stored user noise signature data may indicate one or more user noise thresholds, and the user noise signal may be determined as meeting the criteria if the comparison indicates that the user noise signal is below or within the one or more user noise thresholds.

The stored user noise signature data may be a user noise learned model, generated using one or more prior training operations, for classifying whether or not the received user noise signal is below or within the one or more noise thresholds.

The apparatus may further comprise, prior to receiving the control signal, means for performing training to generate the user noise learned model, the training may comprise means for receiving from the user a plurality of control signals corresponding to operation of the remote apparatus, means for extracting a plurality of user noise signals corresponding to each training control signal, and means for generating the learned model iteratively at least partially based on the plurality of user noise signals.

The means for extracting the user noise signal from the control signal may comprise means for subtracting a command signal from the control signal, wherein the command signal is configured to control the remote apparatus.

The command signal may be a command signal learned model, generated using one or more prior training operations, for classifying whether or not the received command signal is below or within one or more command thresholds.

The apparatus may further comprise, prior to receiving the control signal, means for performing training to generate the command signal learned model, the training may comprise means for receiving from the user a plurality of control signals corresponding to operation of the remote apparatus, means for extracting a plurality of command signals corresponding to each training control signal, and means for generating the learned model iteratively at least partially based on the plurality of command signals.

The command signal may be determined by filtering out noise from the control signal.

The noise may include the user noise signal and a hardware noise signal, wherein the hardware noise signal corresponds to the control apparatus.

The predetermined criteria may be met at least partially based on the probability that the user noise signal is the same as a stored noise signature is above a predetermined threshold.

The authenticating may comprise means for authenticating the user of the control apparatus to enable operation of the apparatus by means of the control signal if the user noise signal meets the one or more predetermined criteria.

The authenticating may comprise means for preventing user operation of the apparatus by means of the control signal if the user noise signal does not meet the one or more predetermined criteria.

The apparatus may further comprise means for causing disabling of the remote apparatus until a reset event is performed.

The apparatus may further comprise means for causing an alarm to sound at the remote apparatus and/or at a remote computer terminal associated with the remote apparatus.

The determining and authenticating may be performed repeatedly, in substantial real-time, for the control signal received whilst the user is operating the control apparatus.

The remote apparatus may be a robot for being mechanically controlled by the control apparatus.

The robot may be performing a medical procedure.

In a second aspect, the specification describes a method, comprising: receiving a control signal from a user-operated control apparatus for controlling a remote apparatus, wherein the control signal is a composite signal comprising a command signal for controlling the remote apparatus and a user noise signal; extracting the user noise signal from the received control signal by subtracting from the control signal one or more known command signals for possible control actions of the remote apparatus; determining if the user noise signal meets one or more predetermined criteria; and authenticating a user of the control apparatus at least partially based on the user noise signal determination.

The method may further comprise identifying a user from whom the control signal is received, and wherein determining if the user noise signal meets the predetermined criteria includes comparing the user noise signal with stored user noise signature data corresponding to the identified user.

Identifying the user may comprise receiving user-input prior to receiving the control signal.

The received user-input may comprise one or more of a password, fingerprint, or other biometric data.

The stored user noise signature data may indicate one or more user noise thresholds, and wherein the user noise signal is determined as meeting the criteria if the comparison indicates that the user noise signal is below or within the one or more user noise thresholds.

The stored user noise signature data may be a user noise learned model, generated using one or more prior training operations, for classifying whether or not the received user noise signal is below or within the one or more noise thresholds.

The method may further comprise, prior to receiving the control signal, performing training to generate the user noise learned model, the training may comprise receiving from the user a plurality of control signals corresponding to operation of the remote apparatus, extracting a plurality of user noise signals corresponding to each training control signal, and generating the learned model iteratively at least partially based on the plurality of user noise signals.

Extracting the user noise signal from the control signal may comprise subtracting a command signal from the control signal, wherein the command signal is configured to control the remote apparatus.

The command signal may be a command signal learned model, generated using one or more prior training operations, for classifying whether or not the received command signal is below or within one or more command thresholds.

The method may further comprise, prior to receiving the control signal, performing training to generate the command signal learned model, the training may comprise receiving from the user a plurality of control signals corresponding to operation of the remote apparatus, extracting a plurality of command signals corresponding to each training control signal, and generating the learned model iteratively at least partially based on the plurality of command signals.

The command signal may be determined by filtering out noise from the control signal.

The noise may include the user noise signal and a hardware noise signal, wherein the hardware noise signal corresponds to the control apparatus.

The predetermined criteria may be met at least partially based on the probability that the user noise signal is the same as a stored noise signature is above a predetermined threshold.

The authenticating may comprise authenticating the user of the control apparatus to enable operation of the apparatus by means of the control signal if the user noise signal meets the one or more predetermined criteria.

The authenticating may comprise preventing user operation of the apparatus by means of the control signal if the user noise signal does not meet the one or more predetermined criteria.

The method may further comprise causing disabling of the remote apparatus until a reset event is performed.

The method may further comprise causing an alarm to sound at the remote apparatus and/or at a remote computer terminal associated with the remote apparatus.

The determining and authenticating may be performed repeatedly, in substantial real-time, for the control signal received whilst the user is operating the control apparatus.

The remote apparatus may be a robot for being mechanically controlled by the control apparatus.

The robot may be for performing a medical procedure.

In a third aspect, the specification describes an integrated circuit configured to perform any method as described with reference to the second aspect.

The integrated circuit may comprise an application-specific integrated circuit (ASIC).

In a fourth aspect, the specification describes a non-transitory computer-readable storage medium having stored thereon computer-readable code, which, when executed by at least one processor, causes the at least one processor to perform a method, comprising: receiving a control signal from a user-operated control apparatus for controlling a remote apparatus; extracting a user noise signal from the received control signal; determining if the user noise signal meets one or more predetermined criteria; and authenticating a user of the control apparatus at least partially based on the user noise signal determination.

In a fifth aspect, the specification describes an apparatus comprising: at least one processor; and at least one memory including computer program code which, when executed by the at least one processor, causes the apparatus: to receive a control signal from a user-operated control apparatus for controlling a remote apparatus; to extract a user noise signal from the received control signal; to determine if the user noise signal meets one or more predetermined criteria; and to authenticate a user of the control apparatus at least partially based on the user noise signal determination.

In a sixth aspect, the specification describes an apparatus, comprising: means for receiving a control signal from a user; and means for sending the control signal to an authentication apparatus for controlling the apparatus.

The apparatus may be a user-operated control apparatus.

The apparatus may be headset, for example a virtual reality or augmented reality headset.

In a seventh aspect, the specification describes an apparatus comprising: a means for receiving an control signal for controlling the apparatus.

The apparatus may be a remote device, for example a robot.

## Brief Description of Drawings

Example embodiments will now be described, by way of non-limiting example, with reference to the accompanying drawings, in which:

FIG. 1 is a schematic diagram of a system involving user control of a remote device over a network, according to an example embodiment;

FIG. 2 is a graphical representation of how a control signal can be separated into command and noise components authenticating a user, according to an example embodiment;

FIG. 3 is a flow diagram showing operations involved in calibrating or training an authentication system to derive a user noise signature, according to an example embodiment;

FIG. 4 is a flow diagram showing operations involved in authenticating a user based on a comparison with a noise signature;

FIG. 5 is a schematic block diagram of components that may form an authentication system, according to an example embodiment; and

FIGS. 6A and 6B are representational views of non-transitory media for holding computer-readable code that may perform methods, according to an example embodiment.

## Detailed Description

Example embodiments relate to the remote controlling of devices, which includes machine or processor -based authentication of a user controlling a device remotely. In this context, authentication means checking if a user is a valid user and the result can be a positive one if the user is valid or a negative one if the user is not valid.

For example, a user may be located in a first location and a device that the user is required or wishes to operate may be located in a second location which is remote from the first location. The device may be an electro-mechanical device such as a robot for performing a particular task, such as in relation to manufacturing, providing a service and even performing a manual task which may be mission-critical or medically-related, e.g. remote surgery. However, embodiments are not necessarily limited to remote control of a robot.

A user may effect said remote control by means of operating a processing system, such as by means of any one of a computer, laptop, smartphone, tablet computer, digital assistant, smartwatch or virtual reality (VR) or augmented reality (AR) device. Whatever its form, the processing system may provide an input device such as one or more of a joystick, touch-sensitive pad, touch-sensitive screen, or virtualised input whereby one or more sensors or cameras may detect user movement and/or gestures in relation to a virtualised scene in order to generate control signals for transmission to the remote device under control. In general, example embodiments relate to remote control where some user movement is involved, for example hand or arm movement.

It will be appreciated that virtual reality systems provide an intuitive and effective way for users to feel immersed in a captured environment, for example in relation to a environment comprising a remote device. Virtual reality may, for example, involve capturing and transmitting to a user device video data, possibly accompanied by audio data, which is encoded with a spatial percept such that, when rendered at a virtual reality user device, the user feels visually immersed in a three-dimensional virtualised space. Movement of the virtual reality user device and/or associated input device(s) may permit three-dimensional movement (pitch, roll and yaw) of the user or part of their body in relation to the virtualised space to effect interactive control of a remote device. Further, some virtual reality systems may additionally detect translational movement of the user within the virtualised space to provide so-called six degrees of freedom (6D0F) interaction.

Typically, a virtual reality user device will be a headset or goggles worn by a user. The headset or goggles may comprise a plurality of screens. The headset or goggles may further comprise one or more headphones or speakers for transducing audio signals, if provided, to accompany the virtualised space that is presented through the screens. Sensors such as gyroscopes and accelerometers may be comprised within the headset or goggles for sensing and generating signals representative of user motion, such as pitch, roll, yaw and translational movement, if provided. In other examples, user motion is sensed by external sensors, such as by one or more cameras positioned in the real-world space in which the user is situated. In other examples, the user may carry one or more radio tags which, in association with one or more radio tag readers positioned in the real-world space, may determine the current position of the user. The headset or goggles may also carry one or more cameras for sensing a user's gaze direction.

Example embodiments assume the use of a virtual reality user device for controlling an electromechanical robot, through any suitable means where the controlling input is issued remotely. For example, the robot may be used for a mission-critical task, such as assembling or servicing complex machinery, operating within a hazardous environment and/or performing a medical procedure.

With these examples in mind, authentication of a proper controlling user is needed, for example to avoid intervention of an unauthorised other person part-way through controlling input to the robot or other remote device. For example, a proper user may have authenticated themselves using an initial method by means of identifying themselves with an entered username or radio tag or token, and may then have entered a password. Biometrics may be another source of initial authentication. However, if during controlling input the proper user is physically intimidated or struck, e.g. pushed, embodiments provide an advantageous way of detecting such conditions and taking appropriate action. Embodiments also protect against the user becoming unexpectedly incapacitated during operation.

As mentioned, methods for authenticating a proper user ordinarily take place before the user accesses control of the remote device. Generally, the user enters their credentials to access the device in order to be positively authenticated so that operation of the remote device is enabled. Once the operation of the remote device is enabled, further authentication is not usually required.

Example embodiments herein provide a continuous or substantially continuous authentication of a user whilst the device is being operated, which provides an additional layer of security over conventional authentication methods.

The various aspects will be described in detail with reference to the accompanying drawings. Wherever possible, the same reference numbers will be used throughout the drawings to refer to the same or like parts. References made to particular examples and implementations are for illustrative purposes, and are not intended to limit the scope of protection.

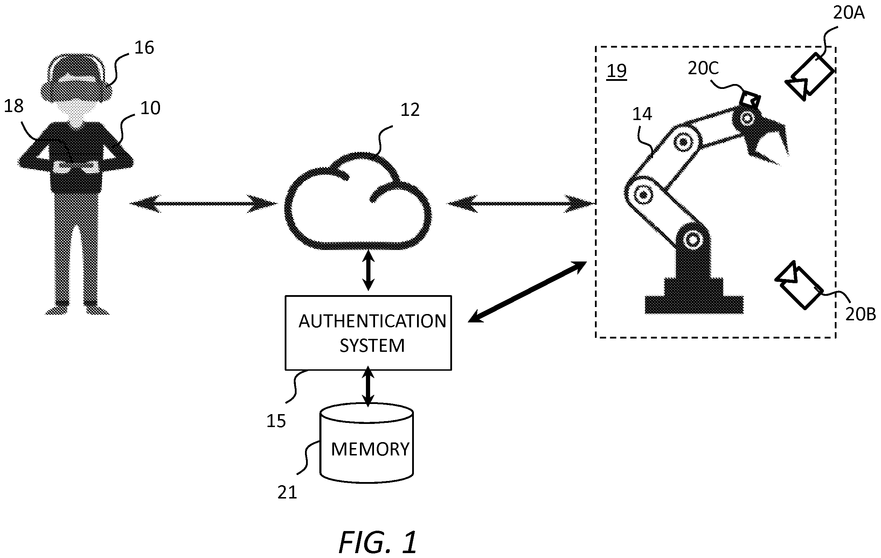

FIG. 1 shows a system including a user 10, a network 12 and a remote device 14. The user 10 may receive from the network 12 (or another network) video data representing a video feed which may be of a virtualised space in which the remote device 14 is located or from a camera attached to the remote device. The video data is received at a processing device such as a computer, media player or the like which may render the video data into a three-dimensional virtualised space which is presented to the user through a virtual reality user device, such as a virtual reality headset (hereafter "headset") 16. In some embodiments, the video data may be rendered by a rendering means of the headset 16 itself. The user 10 may also operate a handheld controller 18 in addition to the virtual reality headset 16. Movement of the user's hand or arm may be sensed either by the virtual reality headset 16 and/or handheld controller 18 and fed-back to the video processing device for transmission over the network 12. This movement may alter the video data that is received subsequently, to reflect motion of the user in relation to the remote device 14 and/or to reflect motion of the remote device as commanded by the user 10. In embodiments herein, it is assumed that the user 10 remains largely static and it is inputs made to the handheld controller 18 that cause the remote device 14 to mechanically move to perform a task.

The FIG. 1 system may also comprise an authentication system 15 configured to authenticate (positively or negatively) the user 10 or other users in relation to operating the remote device 14, in accordance with example embodiments. Although shown as a separate entity, remote from the remote device 14, the authentication system 15 may be located anywhere and may communicate with the user 10 and the remote device 14 via the network 12, or may be local to the user and/or remote device. The authentication system 15 may be provided in the cloud, for example. The authentication system 15 may comprise, or have access to, a memory 21 configured to store data associated with identifiable users. The stored data in said memory 21 may represent user noise signatures relating to their movement during one or more calibration or training operations, performed prior to normal operation.

The headset 16 provides virtual reality video and/or audio content to the user, e.g. through the use of a pair of video screens and/or headphones incorporated within the headset. A virtual space or virtual world is any computer-generated version of a space, for example a captured real world space, in which a user can be immersed through the headset 16.

In example embodiments, the user 10 is immersed in a virtual space 19 that corresponds to the location of the remote device 14. The remote device 14 may have one or more cameras 20A - 20C and/or other sensors to accurately depict a virtual world back to the headset 16.

The network 12 may be any form of network. The network 12 may for example be an Internet Protocol (IP) network such as the Internet. Communication between the headset 16, the handheld controller 18 and the remote device 14 over the network may be by wired or wireless means, such as by means of WiFi or similar, via appropriate gateways, routers and/or other intermediate nodes. Communication may involve radio networks, such as cellular communications networks using 3G, 4G, 5G or future mobile standards.

As the user 10 moves or operates the handheld controller 18, the corresponding movements may be sent as, or converted into, data signals for receipt at the remote device 14. In this way, the user 10 can remotely operate the remote device 14 in real-time or near real-time.

In an example embodiment, the remote device 14 is a robot. For example, the robot 10 may move mechanically in terms of one or more of translation, extension, rotation in the pitch, roll and yaw axes, and to perform open and shutting movements of a pair of jaws at a distal end of an arm. Each type of movement may be commanded by respective control commands which may be issued by the user 10 through the handheld controller 18 in any suitable form, whether as discrete commands resulting from respective hard or soft buttons and/or by means of joystick input and/or by means of touchscreen interaction.

As the user 10 operates the control device 18, a control signal is periodically or continuously generated and transmitted to the remote device 14 via the network 12, causing appropriate movement of the remote device.

The control signal will in practise comprise a composite signal made up of multiple signal components, namely a command signal and one or more noise signals.

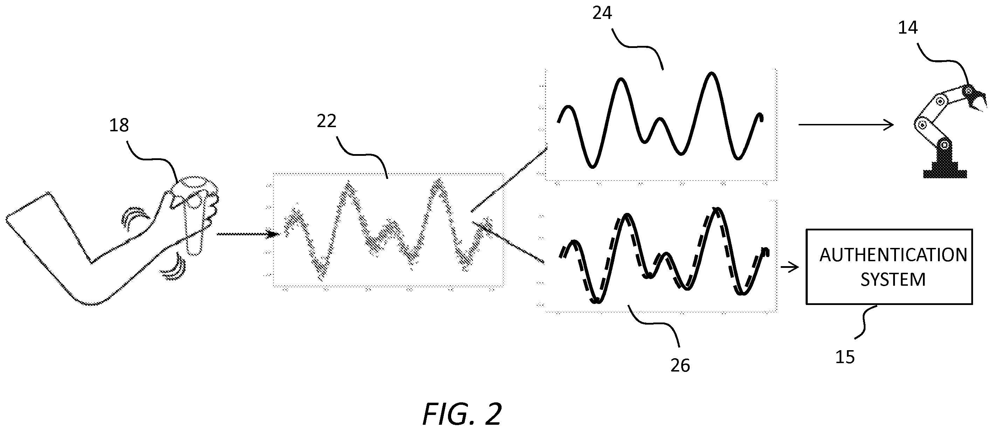

FIG. 2 shows a graphical representation of the control signal 22 which results from operation of the handheld controller 18. The command signal 24 is that component of the composite control signal 22 corresponding to the intended user input through the handheld controller 18; in other words, the component used to provide the intentional instruction to control the remote device 14. The user noise signal 26, shown by a dashed line, is an additional signal component, unique to the user 10 and which may be extracted from the composite control signal 22 using suitable signal processing. The user noise signal 26 is not as a result of intentional commands but due to involuntary movements. Such signal processing may be based on filtering and/or subtracting known command signals for possible commanded control actions from the composite control signal 22 to leave only the user noise signal 26. Other methods may be used.

<>

The user noise signal 26 is therefore also generated by the user 10. For example, the user noise signal 26 may be generated by tremors and/or shaking by the user 10 during normal operation of the handheld controller 18. The user noise signal 26 may therefore be generated by unconscious movements by the user 10, and does not specifically relate to intentional instructions to control the remote device 14. The user noise signal 26 nevertheless represents a form of personal signature of the user, which signature is used herein for periodic or continuous authentication during control of the remote device 14.

In an example embodiment, the composite control signal 22 can be modelled as follows:

$$fx(t) = traj(t) + Eu(t) \qquad\qquad (1)$$

where fx(t) is the composite control signal, traj(t) is the command signal 24 which will be used to operate and control the remote device 14, and Eu(t) is the user noise signal 26, which describes the user's unconscious movements such as tremors and/or shaking.

The above equation (1) may describe the control signal 22 over a single axis. The composite control signal 22 can also be modelled over three axes.

Since the user noise signal Eu(t) 26 will be unique to the user 10, the user noise signal allows the authentication system 15 to continuously check that the user is authorised to operate that remote device 14. This check gives the system an additional layer of security beyond the mere initial authentication based on, e.g. username and password or identity tag. The authorisation check may be continuously performed by the authentication system 15 whilst the remote device 14 is being operated to ensure that the user 10 is authorised to operate the remote device and is not experiencing adverse behaviour such as intimidation or external force, or has become incapacitated.

Prior to operation of the remote device 14, a user noise signal $Eu(t)^T$ may be extracted and stored in the memory 21 of, or associated with, the authentication system 15. This extracting may comprise part of a training or calibration operation. The user 10 may then be given authorisation to operate the remote device 14 and possibly other remote devices within a particular pool of devices, provided the subsequently-received control signal fx(t) 22 during operation comprises a user noise signal component Eu(t) 26 that meets certain conditions associated with the trained user noise signal Eu(t)$^T$ for that user.

For example, the trained user noise signal Eu(t)$^T$ may comprise signature data representing the stored user noise signal Eu(t) from a training or calibration operation. In this context, a signature is simply a data representation of a user's noise signal based on one or more prior training or calibration operations.

For example, the trained user noise signal Eu(t)$^T$ may provide signature data based on an aggregate or average of multiple user noise signals Eu(t) from the training or calibration operation.

For example, the trained user noise signal Eu(t)$^T$ may provide signature data based on a learned model obtained by known machine learning techniques, for example using a neural network, whereby the learned model is trained to classify a received user noise signal Eu(t) during operation into either a positive authentication or a negative authentication.

The trained user noise signal Eu(t)$^T$ is hereinafter referred to as a user noise signature, but it will be appreciated that it can be stored in any suitable form that represents expected user noise, and may be specific to the remote device 14 as well as being user specific.

For example, using any one or more of the above forms of trained user noise signature Eu(t)$^T$, said signature may be converted into a modified signature by one or more processing operations so that it comprises less data than the originally captured user signature during the training or calibration operation. For example, the user noise signature may be reduced to comprise only key frequency components for comparison purposes and/or comprise thresholds defining boundary conditions within which the subsequently-received user noise signal component Eu(t) 26 should remain to result in a positive authentication.

When the user 10 wishes to operate the remote device 14, they may first need to identify themselves to the authentication system 15. This identification may be needed in order to locate the appropriate trained user noise signature Eu(t)$^T$ to access from the memory 21. In use, the user noise signal Eu(t) 26 may be extracted from the control signal fx(t) 22 by filtering or subtraction, and is checked against the trained user noise signature Eu(t)$^T$ stored in the memory 21. If the user noise signal Eu(t) 26 extracted from the control signal fx(t) 22 and the trained user noise signature Eu(t)$^T$ stored in the memory 21 meet one or more predetermined criteria, then positive authentication may result. Any one or more types of criteria may be set. For example, if the user noise signal Eu(t) 26 and the trained user noise signal Eu(t)$^T$ match and/or correlate to an acceptable level, a positive authentication may result and operation of the remote device 14 is enabled or permitted to continue.

In an example embodiment, the user noise signal Eu(t) 26 extracted from the control signal fx(t) 22 and the trained user noise signature Eu(t)$^T$ stored in the memory 21 are tested against one or more criteria based on the probability that the user and the trained user noise signal are the same. The probability that the user and the trained user noise signal are the same may be defined as:

$$P\big(Eu(t)^T = \mathrm{Eu(t)}\big) \triangleq \prod_{t_1}^{t_N} \mathrm{Pr}[Eu(t)^T\big(x(t), y(t), z(t)\big) = \mathrm{Eu(t)}\big(x(t), y(t), z(t)\big)]$$

where $\mathrm{Pr}[Eu(t)^T(x(t),y(t),z(t)) = Eu(t)(x(t),y(t),z(t))]$ is the probability that that three dimensional co-ordinates x(t) , y(t) and z(t) of a user noise signal stored in memory is equal to the user noise signal extracted from the control signal. The product may be taken over a specific time period $[t_1, t_N]$ over which the authentication is run. The time period may be a rolling time window for continuous or periodic authentication. If the probability is above a predetermined threshold, the a positive authentication may result.

In example embodiments, a positive authentication is achieved if the user noise signal Eu(t) 26 extracted from the control signal fx(t) 22 and the trained user noise signature Eu(t)$^T$ stored in the memory correlate to an acceptable level. This may be known as the integral case. The difference between the user noise signal Eu(t) 26 extracted from the control signal fx(t) 22 and the trained user noise signature Eu(t)$^T$ stored in the memory may be defined as:

$$E^{u,u^T}(t_1, t_N) = \int_{t_1}^{t_N} \big|\big|Eu(t)^T - \mathrm{Eu(t)}\big|\big|\, dt$$

$$= \iiint_{x(t),y(t),z(t)} \big|\big|Eu^T(x, y, z) - Eu(x, y, z)\big|\big| dx\, dy\, dt$$

The threshold may be defined as $E_{u^t}^{Abs}$ , above which the delta is too high for the user currently operating the controller to be the user who is represented by user noise signature stored in the memory 21. Therefore, a positive authentication occurs when:

$$\Delta E^{u,u^T}(t_1, t_N) \leq E_{u^t}^{Abs}$$

and a negative authentication occurs when:

$$\Delta E^{u,u^T}(t_1, t_N) > E_{u^t}^{Abs}.$$

If the user noise signal Eu(t) extracted from the control signal fx(t) and the trained user noise signature Eu(t)$^T$ stored in the memory 21 do not match, or do not correlate to an acceptable level, then a negative authentication may result and the user 10 is prevented from operating the remote device 14. In an embodiment, if the user 10 is prevented from operating the remote device 14, the command signal traj(t) 24 is not sent to the remote device therefore ceasing its operation. In an example embodiment, the remote device 14 may be disabled until a reset event is performed. In an example embodiment, an alarm sound may be raised at the remote device 14 and/or at the handheld controller 18.

As mentioned, in an embodiment, a stored user noise signature Eu(t)$^T$ in the memory 21 may indicate one or more user noise thresholds. If the user noise signature Eu(t) 26 is within the user noise signature thresholds, then the user may be positively authenticated to operate the remote device 14. If the user noise signal Eu(t) 26 is not within the user noise signature thresholds, then the user may not be authenticated to operate the remote device 14. This may result in the consequences mentioned above, namely disabling operation of the remote device 14, possibly until a reset event is performed, and possibly raising an alarm.

As also mentioned, the user noise signature Eu(t)$^T$ may use a neural network to produce a learned model that is generated using one or more user prior training operations. The training operations may include receiving a plurality of training control signals from the user, and extracting a plurality of user noise signals from the plurality of control signals and generating the user noise learned model iteratively based on the plurality of user noise signals.

The command signal is a command signal learned model that is generated using one or more prior training operations. The training operations include receiving from the user a plurality of control signals corresponding to operation of the remote device, extracting a plurality of command signals and generating the command signal learned model iteratively based on the plurality of command signals.

In another example embodiment, the control signal can be modelled as follows.

$$fx(t) = traj(t) + Eu(t) + Eh(t)$$

where fx(t) is the control signal, Traj(t) is the command signal which will be used to operate and control the remote device, Eu(t) is the user noise signal, which describes the users unconscious movements such as tremors (as above) and Eh(t) is a hardware noise signal that is the specific noise associated with the handheld controller 18. This example embodiment therefore may take into account other noise components that may be present in the control signal fx(t).

For some systems, the hardware noise signal Eh(t) can be measured at rest. In example embodiments, the hardware noise signal Eh(t) is an isotropic Gaussian noise, which may be assumed or measured.

The user noise signal Eu(t) is not a Gaussian process of the same parameter as the hardware noise signal Eh(t). Therefore, the Gaussian noise can be removed from the overall noise signal (user noise signal plus the hardware noise signal) Eu(t) + Eh(t) to leave only the user noise signal Eh(t). Otherwise, the process repeats as for the previous embodiment.

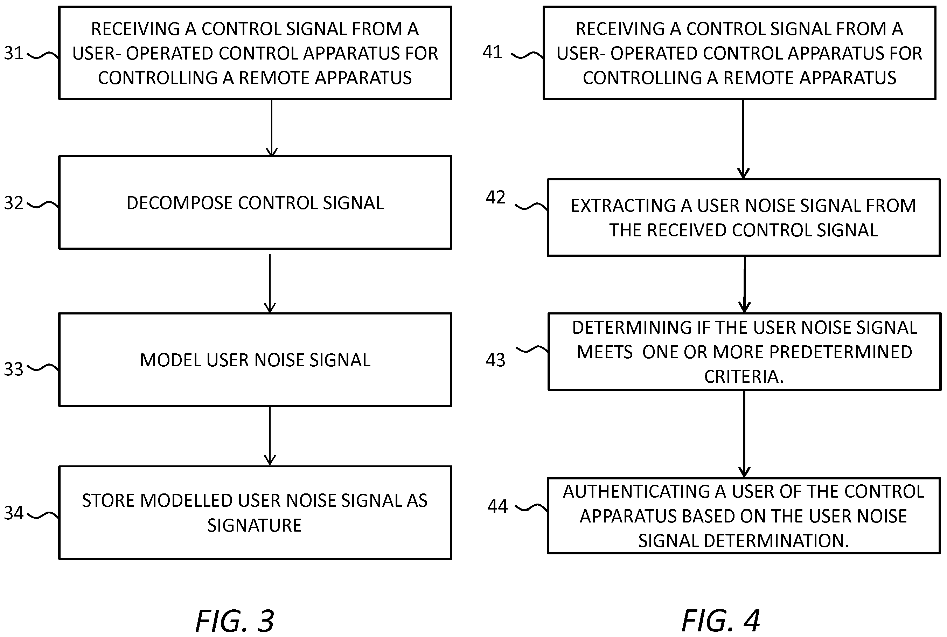

FIG. 3 is a flow diagram representing operations that may be performed by software, hardware, firmware, or a combination thereof as part of a training procedure in embodiments herein. It will be appreciated that further operations may be employed.

A first operation 31 may comprise receiving a control signal from a user-operated control apparatus for controlling a remote apparatus.

Another operation 32 may comprise decomposing the control signal.

Another operation 33 may comprise modelling the user noise signal.

Another operation 34 may comprise storing the user noise signal as a signature for the particular user.

In some embodiments, a plurality of user noise signatures for the same user may be stored for respective remote devices that the user is required to operate, on the basis that different unconscious movements may result from operating different types of remote device. Similarly, different signatures may result from different types of input device. So, whereas a first noise signature may result from operating the remote device 14 using the handheld controller 18, a slightly different second user noise signature may result from operating the remote device 14 using a different handheld controller.

FIG. 4 is a flow diagram representing operations that may be performed by software, hardware, firmware, or a combination thereof as part of an inference or operational procedure in embodiments herein; in other words, when the user is operating the remote device 14. It will be appreciated that further operations may be employed.

A first operation 41 may comprise receiving a control signal from a user-operated control apparatus for controlling a remote apparatus.

Another operation 32 may comprise decomposing the control signal to extract a user noise signal from the received control signal.

Another operation 33 may comprise determining if the user noise signal meets one or more predetermined criteria.

Another operation 34 may comprise authenticating a user of the control apparatus based on the user noise signal determination.

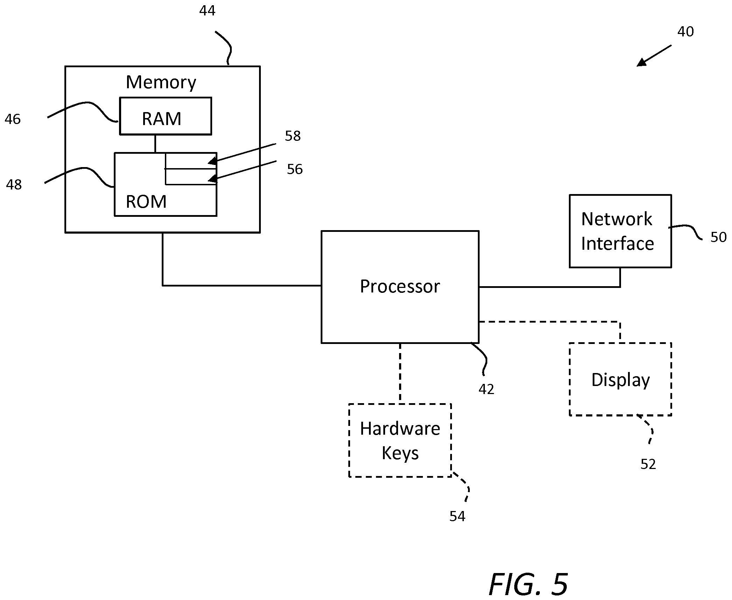

FIG. 5 is a schematic view of an apparatus 40 providing for providing the authentication system 16 above.

The apparatus 40 may have a processor 42, a memory 44 coupled to the processor and comprised or a RAM 46 and ROM 48. The apparatus 40 may comprise a network interface 50, a display 52 and one or more hardware keys 54. The apparatus 40 may comprise one or more such network interfaces 50 for connection to a network, e.g. a radio access network or the network 12 shown in FIG. 1. The one or more network interfaces 50 may also be for connection to the internet, e.g. using WiFi or similar. The processor 42 is connected to each of the other components in order to control operation thereof.

The memory 44 may comprise a non-volatile memory, a hard disk drive (HDD) or a solid state drive (SSD). The ROM 48 of the memory 44 stores, amongst other things, an operating system 56 and may store one or more software applications 58. The RAM 46 of the memory 44 may be used by the processor 42 for the temporary storage of data. The operating system 56 or one or more software applications 58 may contain code which, when executed by the processor, implements the operations as described above, for example with reference to Figures 3 and/or 4.

The processor 42 may take any suitable form. For instance, the processor 42 may be a microcontroller, plural microcontrollers, a processor, or plural processors and the processor may comprise processor circuitry.

Alternatively, the operations described above may be implemented on a dedicated chip or integrated circuit.

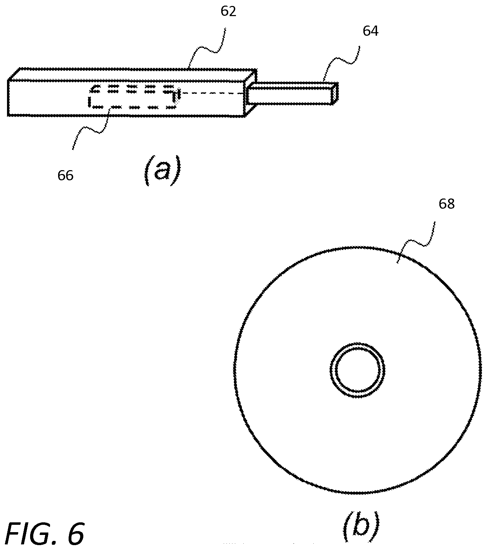

FIG. 6A and FIG. 6B show tangible non-volatile media, respectively a removable memory unit 62 and a compact disc (CD) 68, storing computer-readable code which when run by a computer may perform methods according to embodiments described above. The removable memory unit 62 may be a memory stick, e.g. a USB memory stick, having internal memory 66 storing the computer-readable code. The memory 66 may be accessed by a computer system via a connector 64. The CD 68 may be a CD-ROM or a DVD or similar. Other forms of tangible storage media may be used.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices and other devices. References to computer program, instructions, code etc. should be understood to express software for a programmable processor firmware such as the programmable content of a hardware device as instructions for a processor or configured or configuration settings for a fixed function device, gate array, programmable logic device, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present. As used in this application, references to "based on" may include "at least partially based on."

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a sub-class of the class that includes some but not all of the instances in the class. It is therefore implicitly disclosed that a features described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

**Claims**

1. An apparatus (15), comprising:

   means for receiving a control signal (22) from a user-operated control apparatus (18) for controlling a remote apparatus (14), wherein the control signal is a composite signal comprising a command signal (24) for controlling the remote apparatus and a user noise signal (26);
   means for extracting the user noise signal from the received control signal by subtracting from the control signal one or more known command signals for possible control actions of the remote apparatus;
   means for determining if the user noise signal meets one or more predetermined criteria; and
   means for authenticating a user of the control apparatus at least partially based on the user noise signal determination.

2. The apparatus according to claim 1, wherein the control signal further comprises a hardware noise signal corresponding to noise associated with the user-operated control apparatus and wherein the means for extracting the user noise signal from the received control signal is further configured to subtract from the control signal the hardware noise signal.

3. The apparatus of claim 1 or claim 2, further comprising means for identifying a user from whom the control signal is received, and wherein determining if the user noise signal meets the predetermined criteria includes means for comparing the user noise signal with stored user noise signature data corresponding to the identified user.

4. The apparatus of claim 3, wherein the means for identifying the user comprises means for receiving user-input prior to receiving the control signal.

5. The apparatus of claim 4, wherein the received user-input comprises one or more of a password, fingerprint, or other biometric data.

6. The apparatus of any of claims 3 to 5, wherein the stored user noise signature data indicates one or more user noise thresholds, and wherein the user noise signal is determined as meeting the criteria if the comparison indicates that the user noise signal is below or within the one or more user noise thresholds.

7. The apparatus of claim 6, wherein the stored user noise signature data is a user noise learned model, generated using one or more prior training operations, for classifying whether or not the received user noise signal is below or within the one or more noise thresholds.

8. The apparatus of claim 7, further comprising, prior to receiving the control signal, means for performing training to generate the user noise learned model, the training comprising means for receiving from the user a plurality of control signals corresponding to operation of the remote apparatus, means for extracting a plurality of user noise signals corresponding to each training control signal, and means for generating the learned model iteratively at least partially based on the plurality of user noise signals.

9. The apparatus of any preceding claim, wherein the command signal is a command signal learned model, generated using one or more prior training operations, for classifying whether or not the received command signal is below or within one or more command thresholds.

10. The apparatus of claim 9, further comprising, prior to receiving the control signal, means for performing training to generate the command signal learned model, the training comprising means for receiving from the user a plurality of control signals corresponding to operation of the remote apparatus, means for extracting a plurality of command signals corresponding to each training control signal, and means for generating the learned model iteratively at least partially based on the plurality of command signals.

11. The apparatus of claim 9, wherein the command signal is determined by filtering out noise from the control signal.

12. The apparatus of claim 11, wherein the noise includes the user noise signal and a hardware noise signal, wherein the hardware noise signal corresponds to the control apparatus.

13. The apparatus of any preceding claim, wherein the predetermined criteria is or are met at least partially based on the probability that the user noise signal is the same as a stored noise signature is above a predetermined threshold.

14. A method, comprising:

receiving (41) a control signal from a user-operated control apparatus for controlling a remote apparatus, wherein the control signal is a composite signal comprising a command signal for controlling the remote apparatus and a user noise signal;
extracting (42) the user noise signal from the received control signal by subtracting from the control signal one or more known command signals for possible control actions of the remote apparatus;
determining (43) if the user noise signal meets one or more predetermined criteria; and
authenticating (44) a user of the control apparatus at least partially based on the user noise signal determination.

15. The method according to claim 14, wherein the control signal further comprises a hardware noise signal corresponding to noise associated with the user-operated control apparatus and wherein extracting the user noise signal from the received control signal is further configured to subtract from the control signal the hardware noise signal.


**Patentansprüche**

1. Vorrichtung (15), die Folgendes umfasst:

Mittel zum Empfangen eines Steuersignals (22) von einer von einem Benutzer betriebenen Steuervorrichtung (18) zum Steuern einer entfernten Vorrichtung (14), wobei das Steuersignal ein zusammengesetztes Signal ist, das ein Befehlssignal (24) zum Steuern der entfernten Vorrichtung und ein Benutzerrauschsignal (26) umfasst;
Mittel zum Extrahieren des Benutzerrauschsignals aus dem empfangenen Steuersignal durch Subtrahieren von einem oder mehreren bekannten Befehlssignalen für mögliche Steueraktionen der entfernten Vorrichtung vom Steuersignal;
Mittel zum Bestimmen, ob das Benutzerrauschsignal ein oder mehrere vorbestimmte Kriterien erfüllt; und
Mittel zum Authentifizieren eines Benutzers der Steuervorrichtung mindestens teilweise auf Basis der Benutzerrauschsignalbestimmung.

2. Vorrichtung nach Anspruch 1, wobei das Steuersignal ferner ein Hardwarerauschsignal umfasst, das einem Rauschen entspricht, das mit der vom Benutzer betriebenen Steuervorrichtung verknüpft ist, und wobei die Mittel zum Extrahieren des Benutzerrauschsignals aus dem empfangenen Steuersignal ferner dazu ausgelegt sind, das Hardwarerauschsignal vom Steuersignal zu subtrahieren.

3. Vorrichtung nach Anspruch 1 oder Anspruch 2, die ferner Mittel zum Identifizieren eines Benutzers, von dem das Steuersignal empfangen wird, umfasst, und wobei das Bestimmen, ob das Benutzerrauschsignal die vorbestimmten Kriterien erfüllt, Mittel zum Vergleichen des Benutzerrauschsignals mit gespeicherten Benutzerrauschensignaturdaten, die dem identifizierten Benutzer entsprechen, beinhaltet.

4. Vorrichtung nach Anspruch 3, wobei die Mittel zum Identifizieren des Benutzers vor dem Empfangen des Steuersignals Mittel zum Empfangen einer Benutzereingabe umfassen.

5. Vorrichtung nach Anspruch 4, wobei die empfangene Benutzereingabe eines oder mehreres von einem Passwort, einem Fingerabdruck oder anderen biometrischen Daten umfasst.

**6.** Vorrichtung nach einem der Ansprüche 3 bis 5, wobei die gespeicherten Benutzerrauschensignaturdaten einen oder mehrere Benutzerrauschenschwellwerte anzeigen, und wobei das Benutzerrauschsignal als die Kriterien erfüllend bestimmt wird, wenn der Vergleich anzeigt, dass das Benutzerrauschsignal unter oder innerhalb des einen oder der mehreren Benutzerrauschenschwellwerte liegt.

**7.** Vorrichtung nach Anspruch 6, wobei die gespeicherten Benutzerrauschensignaturdaten ein Benutzerrauschenlernmodell, das unter Verwendung von einer oder mehreren vorherigen Trainingsoperationen erzeugt wird, zum Klassifizieren, ob das empfangene Benutzerrauschsignal unter oder innerhalb des einen oder der mehreren Rauschschwellwerte liegt, ist.

**8.** Vorrichtung nach Anspruch 7, die vor dem Empfangen des Steuersignals ferner Mittel zum Durchführen eines Trainings umfasst, um das Benutzerrauschenlernmodell zu erzeugen, wobei das Training Mittel zum Empfangen einer Vielzahl von Steuersignalen, die dem Betrieb der entfernten Vorrichtung entsprechen, vom Benutzer, Mittel zum Extrahieren einer Vielzahl von Benutzerrauschsignalen, die jedem Trainingssteuersignal entsprechen, und Mittel zum wiederholten Erzeugen des Lernmodells mindestens teilweise auf Basis der Vielzahl von Benutzerrauschsignalen umfasst.

**9.** Vorrichtung nach einem der vorhergehenden Ansprüche, wobei das Befehlssignal ein Befehlssignallernmodell, das unter Verwendung von einer oder mehreren vorherigen Trainingsoperationen erzeugt wird, zum Klassifizieren, ob das empfangene Befehlssignal unter oder innerhalb von einem oder mehreren Befehlsschwellwerten liegt, ist.

**10.** Vorrichtung nach Anspruch 9, die vor dem Empfangen des Steuersignals ferner Mittel zum Durchführen eines Trainings umfasst, um das Befehlssignallernmodell zu erzeugen, wobei das Training Mittel zum Empfangen einer Vielzahl von Steuersignalen, die dem Betrieb der entfernten Vorrichtung entsprechen, vom Benutzer, Mittel zum Extrahieren einer Vielzahl von Befehlssignalen, die jedem Trainingssteuersignal entsprechen, und Mittel zum wiederholten Erzeugen des Lernmodells mindestens teilweise auf Basis der Vielzahl von Befehlssignalssignalen umfasst.

**11.** Vorrichtung nach Anspruch 9, wobei das Befehlssignal durch Herausfiltern von Rauschen aus dem Steuersignal bestimmt wird.

**12.** Vorrichtung nach Anspruch 11, wobei das Rauschen das Benutzerrauschsignal und ein Hardwarerauschsignal beinhaltet, wobei das Hardwarerauschsignal der Steuervorrichtung entspricht.

**13.** Vorrichtung nach einem der vorhergehenden Ansprüche, wobei die vorbestimmten Kriterien mindestens teilweise darauf basierend, dass die Wahrscheinlichkeit, dass das Benutzerrauschsignal dasselbe ist wie eine gespeicherte Rauschsignatur, über einem vorbestimmten Schwellwert liegt, erfüllt sind.

**14.** Verfahren, das Folgendes umfasst:

Empfangen (41) eines Steuersignals von einer von einem Benutzer betriebenen Steuervorrichtung zum Steuern einer entfernten Vorrichtung, wobei das Steuersignal ein zusammengesetztes Signal ist, das ein Befehlssignal zum Steuern der entfernten Vorrichtung und ein Benutzerrauschsignal umfasst;
Extrahieren (42) des Benutzerrauschsignals aus dem empfangenen Steuersignal durch Subtrahieren von einem oder mehreren bekannten Befehlssignalen für mögliche Steueraktionen der entfernten Vorrichtung vom Steuersignal;
Bestimmen (43), ob das Benutzerrauschsignal ein oder mehrere vorbestimmte Kriterien erfüllt; und
Authentifizieren (44) eines Benutzers der Steuervorrichtung mindestens teilweise auf Basis der Benutzerrauschsignalbestimmung.

**15.** Verfahren nach Anspruch 14, wobei das Steuersignal ferner ein Hardwarerauschsignal umfasst, das einem Rauschen entspricht, das mit der vom Benutzer betriebenen Steuervorrichtung verknüpft ist, und wobei das Extrahieren des Benutzerrauschsignals aus dem empfangenen Steuersignal ferner dazu ausgelegt ist, das Hardwarerauschsignal vom Steuersignal zu subtrahieren.

**Revendications**

1. Appareil (15), comprenant :

   des moyens pour recevoir un signal de commande (22) d'un appareil de commande actionné par un utilisateur (18) pour commander un appareil distant (14), dans lequel le signal de commande est un signal composite comprenant un signal d'instruction (24) pour commander l'appareil distant et un signal de bruit d'utilisateur (26) ;
   des moyens pour extraire le signal de bruit d'utilisateur du signal de commande reçu en soustrayant du signal de commande un ou plusieurs signaux d'instruction connus pour des actions de commande possibles de l'appareil distant ;
   des moyens pour déterminer si le signal de bruit d'utilisateur satisfait à un ou plusieurs critères prédéterminés ; et
   des moyens pour authentifier un utilisateur de l'appareil de commande en se basant au moins en partie sur la détermination du signal de bruit d'utilisateur.

2. Appareil selon la revendication 1, dans lequel le signal de commande comprend en outre un signal de bruit matériel correspondant au bruit associé à l'appareil de commande actionné par un utilisateur, et dans lequel les moyens pour extraire le signal de bruit d'utilisateur du signal de commande reçu sont en outre configurés pour soustraire le signal de bruit matériel du signal de commande.

3. Appareil selon la revendication 1 ou la revendication 2, comprenant en outre des moyens pour identifier un utilisateur dont le signal de commande est reçu, et dans lequel la détermination précisant si le signal de bruit d'utilisateur satisfait aux critères prédéterminés comporte des moyens pour comparer le signal de bruit d'utilisateur avec des données de signature de bruit d'utilisateur stockées correspondant à l'utilisateur identifié.

4. Appareil selon la revendication 3, dans lequel les moyens pour identifier l'utilisateur comprennent des moyens pour recevoir une entrée utilisateur avant de recevoir le signal de commande.

5. Appareil selon la revendication 4, dans lequel l'entrée utilisateur reçue comprend un ou plusieurs parmi un mot de passe, une empreinte digitale, ou d'autres données biométriques.

6. Appareil selon l'une des revendications 3 à 5, dans lequel les données de signature de bruit d'utilisateur stockées indiquent un ou plusieurs seuils de bruit d'utilisateur, et dans lequel le signal de bruit d'utilisateur est déterminé comme satisfaisant aux critères si la comparaison indique que le signal de bruit d'utilisateur est en dessous ou à l'intérieur des un ou plusieurs seuils de bruit d'utilisateur.

7. Appareil selon la revendication 6, dans lequel les données de signature de bruit d'utilisateur stockées sont un modèle appris de bruit d'utilisateur généré à l'aide d'une ou plusieurs opérations d'apprentissage préalables, pour effectuer un classement selon que le signal de bruit d'utilisateur reçu est ou non en dessous ou à l'intérieur des un ou plusieurs seuils de bruit.

8. Appareil selon la revendication 7, comprend en outre, avant de recevoir le signal de commande, des moyens pour réaliser un entraînement pour générer le modèle appris de bruit d'utilisateur, l'entraînement comprenant des moyens pour recevoir de l'utilisateur une pluralité de signaux de commande correspondant au fonctionnement de l'appareil distant, des moyens pour extraire une pluralité de signaux de bruit d'utilisateur correspondant à chaque signal de commande d'entraînement, et des moyens pour générer le modèle appris de manière itérative en se basant au moins en partie sur la pluralité de signaux de bruit d'utilisateur.

9. Appareil selon l'une des revendications précédentes, dans lequel le signal d'instruction est un modèle appris de signal d'instruction généré à l'aide d'une ou plusieurs opérations d'apprentissage préalables, pour effectuer un classement selon que le signal d'instruction reçu est ou non en dessous ou à l'intérieur d'un ou plusieurs seuils d'instruction.

10. Appareil selon la revendication 9, comprenant en outre, avant de recevoir le signal de commande, des moyens pour réaliser un entraînement pour générer le modèle appris de signal d'instruction, l'entraînement comprenant des moyens pour recevoir de l'utilisateur une pluralité de signaux de commande correspondant au fonctionnement de l'appareil distant, des moyens pour extraire une pluralité de signaux d'instruction correspondant à chaque signal de commande d'entraînement, et des moyens pour générer le modèle appris de manière itérative en se basant au moins en partie sur la pluralité de signaux d'instruction.

**11.** Appareil selon la revendication 9, dans lequel le signal d'instruction est déterminé en filtrant le bruit du signal de commande.

**12.** Appareil selon la revendication 11, dans lequel le bruit comporte le signal de bruit d'utilisateur et un signal de bruit matériel, dans lequel le signal de bruit matériel correspond à l'appareil de commande.

**13.** Appareil selon l'une des revendications précédentes, dans lequel les critères prédéterminés sont satisfaits en se basant au moins en partie sur la probabilité que le signal de bruit d'utilisateur soit le même lorsqu'une signature de bruit stockée est au-dessus d'un seuil prédéterminé.

**14.** Procédé, comprenant les étapes suivantes :

recevoir (41) un signal de commande d'un appareil de commande actionné par un utilisateur pour commander un appareil distant, dans lequel le signal de commande est un signal composite comprenant un signal d'instruction pour commander l'appareil distant et un signal de bruit d'utilisateur ;
extraire (42) le signal de bruit d'utilisateur du signal de commande reçu en soustrayant du signal de commande un ou plusieurs signaux d'instruction connus pour des actions de commande possibles de l'appareil distant ;
déterminer (43) si le signal de bruit d'utilisateur satisfait à un ou plusieurs critères prédéterminés ; et
authentifier (44) un utilisateur de l'appareil de commande en se basant au moins en partie sur la détermination du signal de bruit d'utilisateur.

**15.** Procédé selon la revendication 14, dans lequel le signal de commande comprend en outre un signal de bruit matériel correspondant au bruit associé à l'appareil de commande actionné par un utilisateur, et dans lequel l'extraction du signal de bruit d'utilisateur du signal de commande reçu est en outre configurée pour soustraire le signal de bruit matériel du signal de commande.

*FIG. 1*

FIG. 2

EP 3 853 749 B1

| | |
|---|---|
| 31 | RECEIVING A CONTROL SIGNAL FROM A USER- OPERATED CONTROL APPARATUS FOR CONTROLLING A REMOTE APPARATUS |
| 32 | DECOMPOSE CONTROL SIGNAL |
| 33 | MODEL USER NOISE SIGNAL |
| 34 | STORE MODELLED USER NOISE SIGNAL AS SIGNATURE |

*FIG. 3*

| | |
|---|---|
| 41 | RECEIVING A CONTROL SIGNAL FROM A USER- OPERATED CONTROL APPARATUS FOR CONTROLLING A REMOTE APPARATUS |
| 42 | EXTRACTING A USER NOISE SIGNAL FROM THE RECEIVED CONTROL SIGNAL |
| 43 | DETERMINING IF THE USER NOISE SIGNAL MEETS ONE OR MORE PREDETERMINED CRITERIA. |
| 44 | AUTHENTICATING A USER OF THE CONTROL APPARATUS BASED ON THE USER NOISE SIGNAL DETERMINATION. |

*FIG. 4*

*FIG. 5*

62

64

66

(a)

68

(b)

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016127006 A
- US 2007094208 A

- US 2017361468 A

**Non-patent literature cited in the description**

- **OROZCO, M. et al.** Automatic identification of participants in haptic systems. *IEEE Instrumentation and Measurement Technology Conference Proceedings*, 16 May 2005, 888-892